# EUROPÄISCHE PATENTSCHRIFT

(11) **EP 2 825 249 B1**
(45) Veröffentlichungstag und Bekanntmachung des Hinweises auf die Patenterteilung: **13.12.2017**
(21) Anmeldenummer: 13720775.9
(22) Anmeldetag: 14.03.2013
(51) Int. Cl.: A61M 39/10, A61M 1/36, A61M 1/16

(54) **MEDIZINISCHE BEHANDLUNGSVORRICHTUNG UND VORRICHTUNG ZUR BEREITSTELLUNG VON MEDIZINISCHEN BEHANDLUNGSFLÜSSIGKEITEN SOWIE VORRICHTUNG ZUM BEFÜLLEN EINER VORRICHTUNG ZUR BEREITSTELLUNG VON MEDIZINISCHEN FLÜSSIGKEITEN**
MEDICAL TREATMENT DEVICE AND DEVICE FOR PROVIDING MEDICAL TREATMENT LIQUIDS, AND DEVICE FOR FILLING A DEVICE FOR PROVIDING MEDICAL LIQUIDS
DISPOSITIF DE TRAITEMENT MÉDICAL ET DISPOSITIF DE FOURNITURE DE LIQUIDES DE TRAITEMENT MÉDICAL, ET DISPOSITIF DE REMPLISSAGE D'UN DISPOSITIF AUX FINS DE FOURNITURE DE LIQUIDES MÉDICAUX

(30) Priorität: 16.03.2012 DE 102012005194; 16.03.2012 US 201261611623 P
(43) Veröffentlichungstag der Anmeldung: 21.01.2015
(73) Patentinhaber: Fresenius Medical Care Deutschland GmbH, 61352 Bad Homburg (DE)
(72) Erfinder: BRANDL, Matthias, 97631 Bad Königshofen (DE); FAULHABER, Thomas, 97493 Bergrheinfeld (DE); HÖRMANN, Jörn, 66386 St. Ingbert (DE); KUGELMANN, Franz, 66606 St. Wendel/Bliesen (DE); ÖRTER, Gökhan, 35789 Weilmünster (DE); STERZER, Rafael, 97422 Schweinfurt (DE)
(74) Vertreter: Oppermann, Frank
(86) Internationale Anmeldenummer: PCT/EP2013/000775
(87) Internationale Veröffentlichungsnummer: WO 2013/135389

(56) Entgegenhaltungen:
- EP-A1- 1 912 131
- WO-A1-2009/144726
- US-A1- 2005 154 368
- US-A1- 2009 012 449

## Beschreibung

Die Erfindung betrifft ein medizinisches Behandlungssystem mit mindestens einer Vorrichtung zur Bereitstellung einer medizinischen Flüssigkeit für eine extrakorporale Dialysevorrichtung oder eine Vorrichtung zur Peritonealdialyse, mit mindestens einer extrakorporalen Dialysevorrichtung oder Vorrichtung zur Peritonealdialyse, mit mindestens einer Vorrichtung (3) zur Befüllung der Vorrichtung zur Bereitstellung von einer medizinischen Flüssigkeit.

Zum Anschluss von externen Komponenten an medizintechnische Einrichtungen ist eine Vielzahl von Konnektoren bekannt. Der Zugang zu den medizintechnischen Einrichtungen erfolgt im Allgemeinen mittels Steckern, die in passende Buchsen der medizintechnischen Einrichtungen eingesteckt werden. Insofern verfügen die medizintechnischen Einrichtungen, die nachfolgend als medizinische Vorrichtungen bezeichnet werden, über eine entsprechende Buchsen-Einheit, während die externen Komponenten eine Stecker-Einheit aufweisen.

Zur Behandlung von nierenkranken Patienten finden Blutbehandlungsvorrichtungen Verwendung, zu denen insbesondere die bekannten extrakorporalen Dialysevorrichtungen oder Vorrichtungen zur Peritonealdialyse gehören. Zur Blutreinigung des Patienten ist die Bereitstellung von medizinischen Behandlungsflüssigkeiten erforderlich. Zu diesen zählen beispielsweise die Dialysierflüssigkeit oder Substitutionsflüssigkeit. In der sogenannten Automatic Peritoneal Dialysis (APD) oder der Akutdialyse werden die medizinischen Behandlungsflüssigkeiten in den Blutbehandlungsvorrichtungen automatisch verarbeitet. Die Behandlungsflüssigkeiten werden in Flüssigkeitsreservoirs bereitgestellt, die an die Behandlungsvorrichtungen angeschlossen werden. Die frische Dialysierflüssigkeit wird aus dem Flüssigkeitsreservoir in die Blutbehandlungsvorrichtung und verbrauchte Flüssigkeit aus der Behandlungsvorrichtung in das Flüssigkeitsreservoir gepumpt. Das Flüssigkeitsreservoir kann bereits ein Konzentrat enthalten, das mit Wasser verdünnt wird. In diesem Fall muss das Flüssigkeitsreservoir nur mit Wasser befüllt werden. Daher wird in diesem Zusammenhang auch Wasser als medizinische Flüssigkeit verstanden. Es ist auch möglich, dass mehrere Flüssigkeitsreservoirs mit einer Blutbehandlungsvorrichtung verbunden sind, wenn in der Behandlungsvorrichtung eine gebrauchsfertige Behandlungsflüssigkeit durch Mischen mehrerer Flüssigkeiten hergestellt wird. Der Anschluss der Flüssigkeitsreservoirs an die Blutbehandlungsvorrichtungen erfolgt wieder mit einer Stecker-Einheit, die in eine Buchsen-Einheit der Blutbehandlungsvorrichtung eingesteckt wird.

Zum Befüllen der Vorrichtungen zur Bereitstellung von Dialyseflüssigkeit sind Vorrichtungen bekannt, an die sich die Vorrichtungen zur Bereitstellung von Dialysierflüssigkeit anschließen lassen. Dafür verfügen die Vorrichtungen zum Befüllen wiederum über eine Buchsen-Einheit, die sich mit der Stecker-Einheit der Vorrichtung zur Bereitstellung von Dialysierflüssigkeit konnektieren lässt.

Eine Vorrichtung zur Bereitstellung einer Behandlungsflüssigkeit ist beispielsweise aus der EP 0 575 970 A2 bekannt. Die bekannte Vorrichtung zur Bereitstellung von Dialysierflüssigkeit umfasst einen Beutel zur Aufnahme der Flüssigkeit, an dem eine Schlauchleitung angeschlossen ist, die an ihrem freien Ende mit einem Stecker verbunden ist. Die Dialysevorrichtung verfügt über eine Buchse, in die der Stecker eingesteckt wird. Mit dem Stecker und der Buchse können zwei Strömungsverbindungen hergestellt werden, um frische Dialysierflüssigkeit aus dem Beutel in die Dialysevorrichtung und verbrauchte Dialysierflüssigkeit zurück in den Beutel leiten zu können.

Bei der Befüllung der Vorrichtung zur Bereitstellung von medizinischen Flüssigkeiten muss sichergestellt sein, dass eine Wiederverwendung der Vorrichtung zur Bereitstellung von medizinischen Flüssigkeiten ausgeschlossen ist.

Die EP 0 476 089 B1 beschreibt eine Vorrichtung zur Spülung von Gewebe mit einem Modul zur Aufnahme einer zur einmaligen Verwendung bestimmten Kassette. Das Modul weist eine hinterschnittene Ausnehmung auf, in der ein hakenförmiger Ansatz der Kassette sitzt, wenn die Kassette in das Modul eingesetzt ist. Wenn die Kassette aus dem Modul herausgezogen wird, bricht der hakenförmige Ansatz ab Aus der EP 0 947 206 ist eine Stecker-Einheit einer Vorrichtung zur Bereitstellung von Dialysierflüssigkeit bekannt, die über einen Strichcode verfügt.

Die US 2005/154368 A1 beschreibt eine Anordnung, die eine erste Spritze zur Entnahme von Blut des Patienten und Zuführen von Blut zu einer Blutbehandlungseinheit, eine zweite Spritze zur Entnahme von behandeltem Blut von der Blutbehandlungseinheit und eine Vorrichtung aufweist, die eine Zuführ- und Entnahmeeinheit und die Blutbehandlungseinheit umfasst. Zuführ- und Entnahmeeinheit und Blutbehandlungseinheit bilden eine bauliche Einheit. Die erste und zweite Spritze weisen jeweils einen RFID-Schaltkreis auf. Die Blutbehandlungseinheit deaktiviert den RFID-Schaltkreis, nachdem die Spritze mit der Zuführ- und Entnahmeeinheit verbunden worden ist. Der RFID-Schaltkreis der Spritze kann somit zwei Betriebszustände signalisieren, d. h. ob die Spritze mit der Zuführ- und Entnahmeeinheit verbunden worden ist oder nicht.

Aus der WO 2009/144726 A1 ist eine Vorrichtung zur Applikation einer medizinischen Flüssigkeit bekannt, die aus einer zur einmaligen Verwendung bestimmten Einheit und einer zur Wiederverwendung bestimmten Einheit besteht. Beide Einheiten werden miteinander verbunden, um dem Patienten eine medizinische Flüssigkeit zuführen zu können. Die eine Einheit ist mit einem Informationsträger (ID Tag) versehen, während die andere Einheit ein ID Lesegerät aufweist. Beim Verbinden beider Einheiten wird der Informationsträger unbrauchbar gemacht. Dadurch soll verhindert werden, dass die Informationen bei einer nicht gewünschten Wiederverwendung der Vorrichtung von dem Informationsträger nicht mehr gelesen werden können. Die WO 2009/144726 A1 beschreibt nur eine zweiteilige Vorrichtung zur Applikation einer medizinischen Flüssigkeit, die nur eine Einheit bildet, wenn die Vorrichtung verwendet wird.

Der Erfindung liegt die Aufgabe zu Grunde, die Sicherheit bei der Versorgung der extrakorporalen Dialysevorrichtungen oder Vorrichtungen zur Peritonealdialyse, mit medizinischen Flüssigkeiten, insbesondere Dialysierflüssigkeit, zu erhöhen.

Die Lösung dieser Aufgabe erfolgt erfindungsgemäß mit den Merkmalen des Patentanspruchs 1. Vorteilhafte Ausführungsformen der Erfindung sind Gegenstand der Unteransprüche.

Die Blutbehandlungsvorrichtung und die Vorrichtung zur Befüllung der Vorrichtung zur Bereitstellung von medizinischen Flüssigkeiten des erfindungsgemäßen medizinischen Behandlungssystems verfügen über eine Buchsen-Einheit, während die Vorrichtung zur Bereitstellung von medizinischen Flüssigkeiten des medizinischen Behandlungssystems über eine Stecker-Einheit verfügt. Die Buchsen-Einheit und die Stecker-Einheit zeichnen sich dadurch aus, dass sich mit beiden Einheiten eine sichere Verbindung zwischen der Vorrichtung zur Bereitstellung von medizinischen Flüssigkeiten und der Blutbehandlungsvorrichtung einerseits oder der Vorrichtung zum Befüllen der Vorrichtung zur Bereitstellung von medizinischen Flüssigkeiten andererseits einfach herstellen lässt.

Die Stecker-Einheit der Vorrichtung zur Bereitstellung von medizinischen Flüssigkeiten weist Mittel zur Signalisierung von zwei Betriebszuständen auf,
während die Buchsen-Einheit der medizinischen Behandlungsvorrichtung Mittel zum Beschädigen oder Zerstören der Mittel zur Signalisierung von zwei Betriebszuständen aufweist. Die Mittel zur Signalisierung von zwei Betriebszuständen und die Mittel zum Beschädigen oder Zerstören der Mittel zur Signalisierung von zwei Betriebszuständen sind derart ausgebildet, dass die Mittel zur Signalisierung von zwei Betriebszuständen beim Anschluss der Stecker-Einheit der Vorrichtung zur Bereitstellung von einer medizinischen Flüssigkeit an die Buchsen-Einheit der medizinischen Behandlungsvorrichtung beschädigt oder zerstört werden. Der erste Betriebszustand ist somit der Zustand vor dem Anschluss der Stecker-Einheit an die Buchseneinheit, wenn die Mittel unversehrt sind, und der zweite Betriebszustand ist der Zustand nach dem Anschluss der Stecker-Einheit an die Buchsen-Einheit ist, wenn die Mittel beschädigt oder zerstört sind. Die Mittel zum Signalisieren von den beiden Betriebszuständen können auch weitere Informationen signalisieren. Entscheidend ist aber, dass diese Mittel zumindest die beiden Betriebszustände anzeigen. Bei einer bevorzugten Ausführungsform werden diese Mittel durch einen Informationsträger gebildet.

Die Mittel zum Signalisieren von den beiden Betriebszuständen brauchen nicht vollständig zerstört werden. Es reicht aus, wenn diese Mittel, falls sie eine Funktion haben, unbrauchbar werden, beispielsweise die Informationen auf dem Informationsträger nicht mehr lesbar sind.

Nach der bestimmungsgemäßen einmaligen Verwendung der Vorrichtung zur Bereitstellung von einer medizinischen Flüssigkeit, d.h. wenn die Flüssigkeit der Behandlungsvorrichtung zugeführt worden ist, kann die Vorrichtung nicht wieder mit Flüssigkeit befüllt werden. Damit ist eine Wiederverwendung der Vorrichtung ausgeschlossen.

Die Befüllung einer bereits verwendeten Vorrichtung zur Bereitstellung von einer medizinischen Flüssigkeit wird mit der erfindungsgemäßen Vorrichtung zur Befüllung der Vorrichtung zur Bereitstellung einer medizinischen Flüssigkeit ausgeschlossen, die eine Buchsen-Einheit zum Anschluss der Stecker-Einheit der Vorrichtung zur Bereitstellung einer medizinischen Flüssigkeit aufweist.

Die Vorrichtung zur Befüllung weist Mittel zur Erkennung der Wiederverwendung der Vorrichtung zur Bereitstellung einer medizinischen Flüssigkeit auf, die derart ausgebildet sind, dass detektiert wird, ob die Mittel zur Signalisierung von zwei Betriebszuständen der Stecker-Einheit der Vorrichtung zur Bereitstellung einer medizinischen Flüssigkeit unversehrt bzw. beschädigt oder zerstört sind.

Das Grundprinzip der Erfindung liegt darin, die Wiederverwendung der Vorrichtung zur Bereitstellung von einer medizinischen Flüssigkeit dadurch zu verhindern, dass bereits die Befüllung der Vorrichtung mit einer medizinischen Flüssigkeit ausgeschlossen ist. Dadurch wird ausgeschlossen, dass Vorrichtungen zur Bereitstellung von medizinischen Flüssigkeiten in Umlauf kommen können, die schon einmal verwendet worden sind.

Bei einer bevorzugten Ausführungsform der Erfindung weisen die Mittel zur Signalisierung von zwei Betriebszuständen einen flächigen Informationsträger auf, der maschinenlesbare Informationen über charakteristische Eigenschaften der medizinischen Flüssigkeit, beispielsweise die Zusammensetzung der Flüssigkeit oder die Flüssigkeitsmenge, trägt. Der Informationsträger dient also nicht nur zur Bereitstellung maschinenlesbarer Informationen, sondern auch zur Erkennung der Wiederverwendung der Vorrichtung. Die Informationen auf dem Informationsträger können beispielsweise ein Matrix-Code, aber auch jeder andere bekannte maschinenlesbare Code sein.

Eine weitere bevorzugte Ausführungsform sieht vor, dass der Informationsträger auf einem Teilstück der Stecker-Einheit der Vorrichtung zur Bereitstellung einer medizinischen Flüssigkeit aufgebracht ist, der mit einer Vertiefung versehen ist, wobei die Mittel zum Beschädigen oder Zerstören des flächigen Informationsträgers der Behandlungsvorrichtung einen vorspringenden Ansatz aufweisen, der beim Anschluss der Stecker-Einheit der Vorrichtung zur Bereitstellung von einer medizinischen Flüssigkeit an die Buchseneinheit der Behandlungsvorrichtung in die Vertiefung greift, so dass der Informationsträger beschädigt oder zerstört und somit unbrauchbar wird.

Bei einer besonders bevorzugten Ausführungsform ist die Vertiefung eine zumindest an einem Ende offene Nut in dem Teilstück der Stecker-Einheit, die parallel zu der Achse der Stecker-Einheit verläuft, in deren Richtung die Stecker-Einheit in die Buchsen-Einheit der Behandlungsvorrichtung eingesteckt wird. Beim Einstecken der Stecker-Einheit in die Buchseneinheit wird der vorspringende Ansatz in die Nut eingeschoben, wodurch der flächige Informationsträger ohne größeren Kraftaufwand sicher zerstört wird.

Die Mittel zur Erkennung der Wiederverwendung der Vorrichtung zur Bereitstellung von einer medizinischen Flüssigkeit weisen vorzugsweise eine Einheit zum Lesen von Informationen auf dem flächigen Informationsträger und eine Auswerteinheit auf, die derart ausgebildet ist, dass ein die Wiederverwendung signalisierendes Signal erzeugt wird, wenn nach der Zerstörung des Informationsträgers die Auslesung von Informationen fehlerhaft ist. Dabei wird unter einem fehlerhaften Auslesen auch verstanden, dass sich die Informationen überhaupt nicht auslesen lassen, was nach einer Zerstörung oder Beschädigung des Informationsträgers der Fall sein dürfte.

Wenn die Vorrichtung zur Befüllung der Vorrichtung zur Bereitstellung von einer medizinischen Flüssigkeit bereits über ein Lesegerät verfügt, um beispielsweise charakteristische Informationen über die Flüssigkeit von einem maschinenlesbaren Code zu lesen, können die Mittel zur Erkennung der Wiederverwendung ohne weiteren gerätetechnischen Aufwand implementiert werden.

Eine weitere besonders bevorzugte Ausführungsform der Vorrichtung zur Befüllung sieht eine Steuereinheit zur Steuerung des Befüllvorgangs vor, die derart ausgebildet ist, dass die Einleitung des Befüllvorgangs verhindert wird, wenn von der Auswerteinheit das die Wiederverwendung signalisierende Signal erzeugt wird.

Die Buchsen-Einheit der Vorrichtung zum Befüllen und die Stecker-Einheit der Vorrichtung zur Bereitstellung von einer medizinischen Flüssigkeit sind vorzugsweise derart ausgebildet, dass bereits die Herstellung einer Strömungsverbindung zwischen der Buchsen-Einheit und der Stecker-Einheit verhindert wird. Es ist aber auch möglich, dass die Förderung der medizinischen Flüssigkeit von der Vorrichtung zur Befüllung in die Vorrichtung zur Bereitstellung der Flüssigkeit verhindert wird.

Zur Herstellung der Strömungsverbindung verfügt die Buchsen-Einheit über mindestens ein Anschlussstück, während die Stecker-Einheit über mindestens einen Konnektor verfügt, wobei sich eine flüssigkeitsdichte Verbindung herstellen lässt, wenn das Anschlussstück an den Konnektor angeschlossen wird. Bei einer bevorzugten Ausführungsform weist die Buchsen-Einheit ein erstes Anschlussstück zum Anschluss eines ersten Konnektors der Stecker-Einheit und ein zweites Anschlussstück zum Anschluss eines zweiten Konnektors der Stecker-Einheit auf, sodass eine erste Strömungsverbindung zum Zuführen frischer Behandlungsflüssigkeit und eine zweite Strömungsverbindung zum Abführen verbrauchter Behandlungsflüssigkeit hergestellt werden kann.

Wenn die Vorrichtung zur Bereitstellung von einer medizinischen Flüssigkeit nicht nur der Bereitstellung frischer Flüssigkeit, sondern auch der Aufnahme verbrauchter Flüssigkeit dient, kann die Vorrichtung zum Befüllen der Vorrichtung zur Bereitstellung von einer medizinischen Flüssigkeit auch zum Entleeren der Vorrichtung zur Bereitstellung von Flüssigkeit dienen. In diesem Fall wird nur der Befüllvorgang mit frischer Flüssigkeit verhindert. Die bereits verwendete Vorrichtung soll aber von der Vorrichtung zum Befüllen noch entleert werden können.

Die Vorrichtung zur Bereitstellung von medizinischen Flüssigkeiten kann im einfachsten Fall ein Kanister oder Beutel sein, der über die Stecker-Einheit verfügt, beispielsweise ein Beutel mit einem Stecker sein.

Im Folgenden wird ein Ausführungsbeispiel der Erfindung unter Bezugnahme auf die Figuren näher erläutert.

Es zeigen:
- Fig. 1:: eine Vorrichtung zur Bereitstellung einer medizinischen Flüssigkeit, insbesondere Dialysierflüssigkeit, zusammen mit einer Blutbehandlungsvorrichtung und einer Vorrichtung zum Befüllen der Vorrichtung zur Bereitstellung von Dialysierflüssigkeit in stark vereinfachter schematischer Darstellung,
- Fig. 2: die Stecker-Einheit der Vorrichtung zur Bereitstellung von Dialysierflüssigkeit zusammen mit der Buchsen-Einheit der Blutbehandlungsvorrichtung von Fig. 1 in perspektivischer Darstellung,
- Fig. 3: die Stecker-Einheit und die Buchsen-Einheit von Fig. 2 in geschnittener Darstellung, wobei Stecker- und Buchsen-Einheit nicht miteinander verbunden sind,
- Fig. 4: die Stecker-Einheit und die Buchseneinheit von Fig. 2 in geschnittener Darstellung, wobei die Buchsen-Einheit zum Anschluss der Stecker-Einheit vorbereitet ist,
- Fig. 5: einen Schnitt durch die Stecker-Einheit und Buchsen-Einheit von Fig. 2, wobei die Stecker-Einheit in die Buchsen-Einheit lose eingesetzt ist, und
- Fig. 6: einen Schnitt durch die Stecker-Einheit und Buchsen-Einheit von Fig. 2, wobei die Stecker-Einheit an die Buchsen-Einheit angeschlossen ist, sodass die Strömungsverbindungen hergestellt sind,
- Fig. 7: die Stecker-Einheit der Vorrichtung zur Bereitstellung von Dialysierflüssigkeit zusammen mit der Buchsen-Einheit der Vorrichtung zum Befüllen der Vorrichtung zur Bereitstellung von Dialysierflüssigkeit in perspektivischer Darstellung,
- Fig. 8A: die Buchsen-Einheit von Fig. 7, die zur Einleitung eines Spülvorgangs vorbereitet ist,
- Fig. 8B: die Buchsen-Einheit von Fig. 7 während des Spülvorgangs,
- Fig. 9: die Stecker-Einheit und Buchsen-Einheit von Fig. 7 in geschnittener Darstellung bevor die Stecker-Einheit und Buchsen-Einheit miteinander verbunden sind,
- Fig. 10: die Stecker-Einheit und Buchsen-Einheit von Fig. 7 in geschnittener Darstellung, wobei die Steckereinheit auf die Buchsen-Einheit lose aufgesetzt ist und
- Fig. 11: einen Schnitt durch die Stecker-Einheit und Buchsen-Einheit von Fig. 7, wobei Stecker-Einheit und Buchsen-Einheit zur Herstellung der Strömungsverbindungen miteinander verbunden sind.

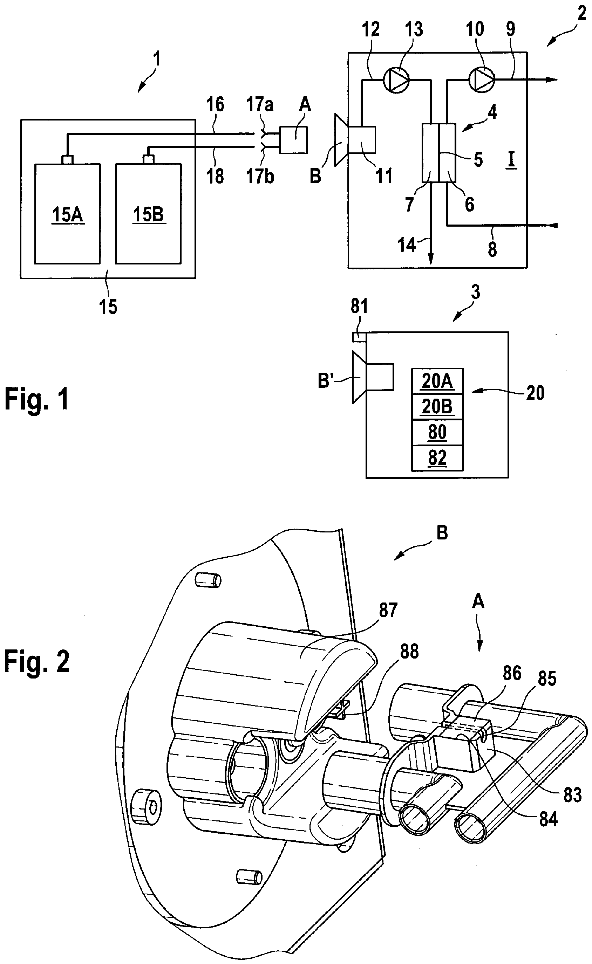

Fig. 1 zeigt in stark vereinfachter schematischer Darstellung eine Vorrichtung 1 zur Bereitstellung einer medizinischen Flüssigkeit, insbesondere Dialysierflüssigkeit, zusammen mit einer Blutbehandlungsvorrichtung 2 und einer Vorrichtung 3 zum Befüllen der Vorrichtung zur Bereitstellung von Dialysierflüssigkeit. Die Blutbehandlungsvorrichtung 2 ist eine extrakorporale Dialysevorrichtung oder eine Vorrichtung zur Peritonealdialyse. Bei dem vorliegenden Ausführungsbeispiel ist die Blutbehandlungsvorrichtung 2 eine Dialysevorrichtung, die über einen Dialysator 4 verfügt, der durch eine semipermeable Membran 5 in eine Blutkammer 6 und eine Dialysierflüssigkeitskammer 7 unterteilt ist. Von dem Patienten führt eine Blutzuführleitung 8 zu der Blutkammer 6 des Dialysators 4, während eine Blutrückführleitung 9, in die eine Blutpumpe 10 geschaltet ist, von der Blutkammer 6 zu dem Patienten führt. Die Blutzuführ- und -rückführleitung, 8, 9 bilden zusammen mit der Blutkammer 6 den extrakorporalen Blutkreislauf I der Dialysevorrichtung 2.

Die frische Dialysierflüssigkeit kann aus einem Dialysierflüssigkeitsreservoir 11 über eine Dialysierflüssigkeitszuführleitung 12, in die eine Dialysierflüssigkeitspumpe 13 geschaltet ist, zu der Dialysierflüssigkeitskammer 7 des Dialysators 4 geleitet werden, während verbrauchte Dialysierflüssigkeit über eine Dialysierflüssigkeitsabführleitung 14 aus der Dialysierflüssigkeitskammer abfließt. Ein Dialysierflüssigkeitsreservoir ist aber nicht erforderlich. Die bereitgestellte Dialysierflüssigkeit kann auch direkt der Dialysierflüssigkeitskammer zugeführt werden.

Zur Bereitstellung frischer Dialysierflüssigkeit dient die Vorrichtung 1, die bei dem vorliegenden Ausführungsbeispiel zwei Beutel oder Kanister 15A und 15B aufweist. Beide Beutel oder Kanister 15A, 15B bilden eine Einheit 15, wobei der Beutel 15A vor der Dialysebehandlung mit frischer Dialysierflüssigkeit befüllt und der Beutel 15B leer ist.

Von dem Dialysierflüssigkeitsbeutel 15A führt eine Zulaufleitung 16 zu dem einen Anschluss 17a einer Stecker-Einheit A, während von dem anderen Anschluss 17b der Stecker-Einheit A eine Ablaufleitung 18 zu dem Leerbeutel 15B führt.

Die Stecker-Einheit A wird zur Bereitstellung von Dialysierflüssigkeit vor der Behandlung an eine Buchsen-Einheit B angeschlossen, die an der Blutbehandlungsvorrichtung 2 vorgesehen ist, sodass frische Dialysierflüssigkeit über die Zulaufleitung 16 dem Dialysierflüssigkeitsreservoir 10 zugeführt und verbrauchte Dialysierflüssigkeit über die Ablaufleitung 18 abgeführt werden kann.

Die Vorrichtung 1 zur Bereitstellung von Dialysierflüssigkeit wird an der Vorrichtung 3 mit frischer Dialysierflüssigkeit befüllt. Mit der Vorrichtung 3 zum Befüllen kann die Vorrichtung 2 zum Bereitstellen von Dialysierflüssigkeit auch entleert werden. Die frische Dialysierflüssigkeit kann in der Vorrichtung 3 zum Befüllen online aus Wasser und Dialysierflüssigkeitskonzentrat hergestellt werden. Frische Dialysierflüssigkeit kann aber auch von einer internen oder externen Dialysierflüssigkeitsquelle zugeführt werden. Verbrauchte Dialysierflüssigkeit kann in einem internen oder externen Reservoir gesammelt oder in einen Ablauf verworfen werden.

Fig. 1 zeigt ein Ausführungsbeispiel, bei dem zur Aufnahme frischer Dialysierflüssigkeit ein Tank 20A und zur Aufnahme verbrauchter Dialysierflüssigkeit ein Tank 20B dient. Die erforderlichen Leitungen und Pumpen werden in der stark schematischen Darstellung nicht gezeigt. Darüber hinaus verfügt die Vorrichtung 1 zur Bereitstellung von Dialysierflüssigkeit über eine Steuereinheit (80), mit der sämtliche Komponenten der Vorrichtung 1 gesteuert werden, um die Vorrichtung zur Bereitstellung von Dialysierflüssigkeit an die Vorrichtung zum Befüllen bzw. Entleeren anzuschließen sowie die Vorrichtung zur Bereitstellung von Dialysierflüssigkeit zu befüllen bzw. zu entleeren, wie im Einzelnen noch beschrieben wird.

Die Vorrichtung 3 zum Befüllen und Entleeren der Vorrichtung 1 zur Bereitstellung frischer und Aufnahme verbrauchter Dialysierflüssigkeit verfügt über eine Buchsen-Einheit B', an die die Stecker-Einheit A der Vorrichtung 1 zur Bereitstellung von Dialysierflüssigkeit angeschlossen wird. Die Buchsen-Einheit B der Blutbehandlungsvorrichtung 2 und die Buchsen-Einheit B' der Vorrichtung zum Befüllen bzw. Entleeren können identisch oder unterschiedlich ausgebildet sein. Bei dem vorliegenden Ausführungsbeispiel sind die Buchsen-Einheiten B bzw. B' unterschiedlich ausgebildet. Beide Buchsen-Einheiten B bzw. B' sind aber derart ausgebildet, dass sich mit der Stecker-Einheit A der Vorrichtung 1 zur Bereitstellung von Dialysierflüssigkeit eine flüssigkeitsdichte Strömungsverbindung mit beiden Vorrichtungen 2 und 3 in beiden Richtungen für frische und verbrauchte Dialysierflüssigkeit herstellen lässt.

Darüber hinaus verfügt die Vorrichtung 3 zum Befüllen bzw. Entleeren über eine Steuereinheit 80 zur Steuerung des Befüll- und Entleervorgangs sowie ein Lesegerät 81 für einen maschinenlesbaren Code von einem flächigen Informationsträger, der an der Stecker-Einheit A der Vorrichtung 1 zur Bereitstellung von Dialysierflüssigkeit vorgesehen ist. Des Weiteren weist die Vorrichtung 3 zum Befüllen bzw. Entleeren eine Auswerteinheit 82 auf, mit der die von dem Informationsträger gelesenen Informationen ausgewertet werden. Die Informationen können beispielsweise Informationen über die Zusammensetzung oder Menge der bereitgestellten medizinischen Flüssigkeit sein. Die Auswerteinheit erlaubt auch eine Fehlererkennung, mit der das fehlerhafte Auslesen eines Codes erkannt wird. Für den Fall, dass die Auslesung des Codes nach einer Zerstörung des Informationsträgers fehlerhaft ist, was auch den Fall einschließt, dass ein Code überhaupt nicht auslesbar ist, erzeugt die Auswerteinheit 82 ein Steuersignal, das die Steuereinheit 80 empfängt. Wenn die Steuereinheit 80 das Steuersignal empfängt, verhindert die Steuereinheit die Einleitung des Befüllvorgangs. Die Einleitung des Befüllvorgangs wird vorzugsweise dadurch verhindert, dass eine Strömungsverbindung zwischen der Stecker-Einheit A der Vorrichtung 1 zur Bereitstellung von Dialysierflüssigkeit und der Buchsen-Einheit der Vorrichtung 3 zum Befüllen bzw. Entleeren nicht automatisch hergestellt wird. Es ist aber auch möglich, dass die an dem Füllvorgang beteiligten nicht dargestellten Pumpen nicht gestartet oder die nicht dargestellten Ventile nicht geöffnet werden.

Nachfolgend wird die Stecker-Einheit A der Vorrichtung 1 zur Bereitstellung von Dialysierflüssigkeit zusammen mit der Stecker-Einheit B der Blutbehandlungsvorrichtung 2 unter Bezugnahme auf die Figuren 2 bis 6 im Einzelnen beschrieben.

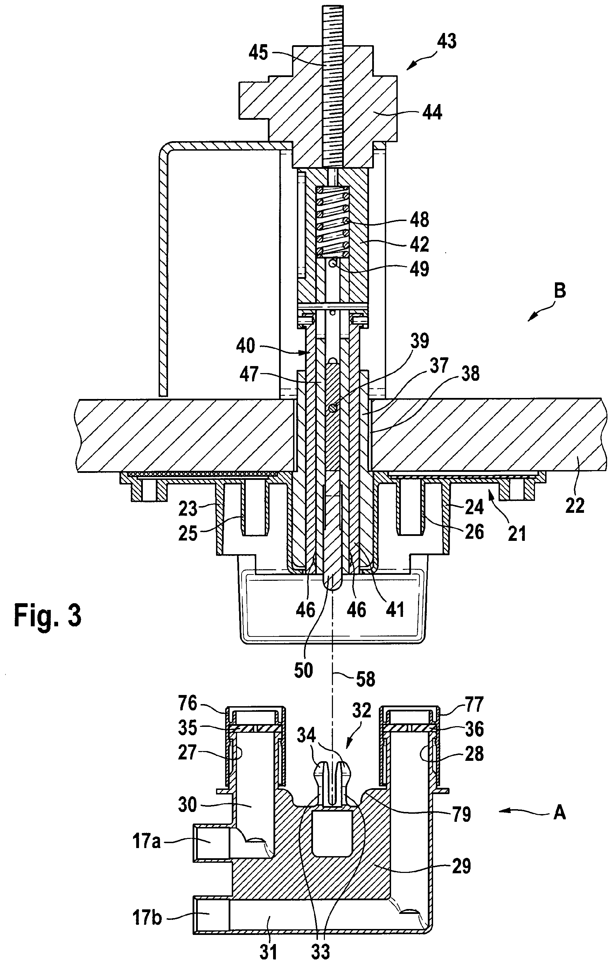

Fig. 2 zeigt die Stecker-Einheit A und die Buchsen-Einheit B in perspektivischer Darstellung, während die Figuren 3 bis 6 die Stecker- und Buchsen-Einheit A, B in geschnittener Darstellung zeigen.

Die Buchsen-Einheit B der Blutbehandlungsvorrichtung 2 ist vorzugsweise Teil einer nicht dargestellten Behandlungskassette, die austauschbar ist. Die Buchsen-Einheit B kann aber auch Teil einer nicht austauschbaren Einheit sein. Die Buchsen-Einheit B weist einen äußeren Flanschteil 21 auf, der mit der Gehäusewand 22 der Behandlungskassette bzw. Blutbehandlungsvorrichtung verbunden oder mit der Wand einstückig sein kann. Beispielsweise kann der äußere Flanschteil 21 mit nicht dargestellten Schrauben mit der Wand verschraubt sein oder mit der Wand ein gemeinsames Spritzgußteil sein. Von dem äußeren Flanschteil 21 stehen zwei zylindrische Anschlussteile 23, 24 ab, die in einer gemeinsamen Ebene zu beiden Seiten der zentralen Achse 58 der Buchsen-Einheit angeordnet sind. Die zylindrischen Anschlussteile 23, 24 umschließen jeweils konzentrisch ein Anschlussstück 25 und 26, wobei das Anschlussstück 25 zum Zuführen frischer Dialysierflüssigkeit und das Anschlussstück 26 zum Abführen verbrauchter Dialysierflüssigkeit dient.

Die Stecker-Einheit A der Vorrichtung 1 zur Bereitstellung frischer und zur Aufnahme verbrauchter Dialysierflüssigkeit verfügt über entsprechende Konnektoren 27, 28, die mit den Anschlussstücken 25, 26 flüssigkeitsdicht verbunden werden. Die Stecker-Einheit A weist einen Steckerkörper 29 auf, der die beiden Konnektoren 27, 28 verbindet. Der Steckerkörper 29 weist einen Zulaufkanal 30 auf, der an dem einen Konnektor 27 angeschlossen ist, und weist einen Ablaufkanal 31 auf, der an dem anderen Konnektor 28 angeschlossen ist. An dem Anschluss 17a des Zulaufkanals 30 ist die Zulaufleitung 16 und an dem Anschluss 17b des Ablaufkanals 31 ist die Ablaufleitung 18 der Vorrichtung 1 zur Bereitstellung von frischer bzw. Aufnahme verbrauchter Dialysierflüssigkeit angeschlossen. Zwischen den beiden Konnektoren 27, 28 befindet sich ein Ansatzstück 32, mit dem eine zunächst nur lose Verbindung zwischen der Stecker-Einheit A und der Buchsen-Einheit B hergestellt werden kann. Das Ansatzstück 32 weist mehrere umfangsmäßig verteilt angeordnete Rastelemente 33 auf, die an einem Ende an dem Stecker-Körper 29 angeformt sind. An den Außenseiten der freien Enden der Rastelemente 33 sind Rastnasen 34 ausgebildet. Die Konnektoren 27 und 28 verfügen über Berührungsschutzhülsen 76 und 77, die auf die Konnektoren 27, 28 des Steckerkörpers 29 einrastend aufgesetzt sind. Die Konnektoren 27, 28 werden jeweils von einem vorzugsweise geschlitzten Septum oder einer Membran 35, 36 verschlossen, die von den Anschlussstücken 25, 26 der Buchsen-Einheit durchdrungen wird.

Zwischen den beiden Konnektoren 27 und 28 weist der Stecker-Körper 29 ein Teilstück 83 auf, an dessen Außenseite 84 sich eine Nut 85 befindet, die von dem vorderen Ende bis zu dem hinteren Ende des Teilstücks 83 parallel zu der Achse verläuft, in deren Richtung die Stecker-Einheit A in die Buchsen-Einheit B eingesteckt wird, so dass die Nut 85 an beiden Enden offenen ist (Fig. 3). Auf das Teilstück 83 ist ein flächiger Informationsträger 86 aufgebracht, der die Nut 85 verdeckt. Der Informationsträger 86 ist ein dünnes Substrat, beispielsweise eine Folie oder Papier, auf das ein Matrix-Code aufgedruckt ist.

Die Buchsen-Einheit B der Blutbehandlungsvorrichtung 2 weist ein nach außen weisendes Gehäusestück 87 auf, das an der Innenseite mit einem vorspringenden Ansatz 88 versehen ist. Die längslaufende Nut 85 in dem Teilstück 83 und der vorspringende Ansatz 88 an dem Gehäusestück 87 sind derart angeordnet, dass beim Einstecken der Stecker-Einheit A in die Buchsen-Einheit B der vorspringende Ansatz 88 in die am vorderen Ende offene Nut 85 greift. Dadurch wird der Informationsträger 86 beim Anschluss der Stecker-Einheit an die Buchsen-Einheit zerstört. Da sich auch der vorspringende Ansatz 88 parallel zu der Achse erstreckt, in deren Richtung die Stecker-Einheit A in die Buchsen-Einheit B eingesteckt wird, dient der Ansatz 88 gleichsam als Führungsstück für die Stecker-Einheit beim Einstecken in die Buchsen-Einheit.

Wenn die Steckereinheit A der nur zum einmaligen Gebrauch bestimmten Vorrichtung 1 zur Bereitstellung frischer und Aufnahme verbrauchter Dialysierflüssigkeit in die Buchsen-Einheit B' der Vorrichtung 3 zum Befüllen bzw. Entleeren angeschlossen wird, kann die Vorrichtung 1 nur entleert, aber nicht wiederbefüllt werden, da der Informationsträger 86 zerstört ist, wie unter Bezugnahme auf Fig. 1 beschrieben ist.

Im Zentrum des Flanschteils 21 der Buchsen-Einheit B sitzt ein zylindrisches Führungsstück 37, dass sich durch eine Bohrung 38 der Gehäusewand 22 erstreckt. In dem Führungsstück 37 ist ein rohrförmiges Aufnahmestück 40 längsverschiebbar geführt, das einen vorderen Abschnitt 41 und einen hinteren Abschnitt 42 aufweist. Zum Verschieben des rohrförmigen Aufnahmestücks 40 in dem zylindrischen Führungsstück 37 ist eine Antriebseinheit 43 vorgesehen. Die Antriebseinheit 43 ist bei dem vorliegenden Ausführungsbeispiel ein elektromotorischer Spindelantrieb, der einen Linearmotor 44 und eine Spindel 45 aufweist, die mit dem hinteren Abschnitt 42 des Aufnahmestücks 40 verbunden ist. Durch Ein- und Ausfahren der Spindel 45 wird das Aufnahmestück 40 aus dem Führungsstück 37 herausgeschoben oder in das Führungsstück zurückgezogen.

An der Innenseite des vorderen Endes des vorderen Abschnitts 41 des Aufnahmestücks 40 sind mehrere umfangsmäßig verteilt angeordnete Ausnehmungen 46 vorgesehen, die derart ausgebildet sind, dass die Rastnasen 34 der Rastelemente 33 des Ansatzstücks 32 der Stecker-Einheit A in die Ausnehmungen 46 einrasten, wenn das Ansatzstück 32 in das Aufnahmestück 40 eingesetzt wird, was nachfolgend noch im Einzelnen beschrieben wird.

Die Buchsen-Einheit B verfügt über Mittel, mit denen erkannt wird, dass das Ansatzstück 32 in das Aufnahmestück 40 eingesetzt ist. Diese Mittel weisen ein als rohrförmiger Körper ausgebildetes Tastelement 47 auf, das längsverschiebbar in dem rohrförmigen Aufnahmestück 40 geführt ist. Das rohrförmige Tastelement 47 ist mit einer Feder 48 vorgespannt, die in dem hinteren Abschnitt 42 des Aufnahmestücks 40 sitzt. Die Bewegung des Tastelements 47 in Längsrichtung wird von einem nur andeutungsweise dargestellten Anschlagelement 49 begrenzt, das in einem in der vorliegenden Schnittebene nicht sichtbaren Schlitz geführt ist, der in dem Aufnahmestück 40 vorgesehen ist.

Zum Detektieren des Ansatzstücks 32 in dem Aufnahmestück 40 verfügt die Buchsen-Einheit B über Mittel, mit denen erkannt wird, ob das vordere Ende des Tastelements 47 bündig mit dem vorderen Ende des Aufnahmestücks 40 abschließt (Fig. 3) oder entgegen der Vorspannung der Feder 48 in das Aufnahmestück zurückgeschoben ist. Dies ist der Fall, wenn das Ansatzstück 32 in das Aufnahmestück 40 eingesetzt ist.

Die Buchsen-Einheit B weist des Weiteren einen stiftförmigen Körper 50 auf, der unbeweglich innerhalb des rohrförmigen Tastelements 47 angeordnet ist. Der stiftförmige Körper 50 kann beispielsweise mit einem Stift 39 arretiert sein, der sich durch in der Schnittebene nicht dargestellte Schlitze des Tastelements 47 und des Aufnahmestücks 40 in das Führungsstück 37 erstreckt.

Nachfolgend wird im Einzelnen beschrieben, wie die Stecker-Einheit A mit der Buchsen-Einheit B verbunden wird.

Fig. 3 zeigt die Ausgangsposition, in der die Buchsen-Einheit B verriegelt ist. In dieser Position ist die Spindel 45 des Linearmotors 43 eingefahren, wodurch das Aufnahmestück 40 mit dem Tastelement 47 zurückgezogen ist, sodass der stiftförmige Körper 50 aus dem Aufnahmestück vorsteht. Der stiftförmige Körper 50 verhindert also das Einsetzen des Ansatzstücks 32 in das Aufnahmestück 40.

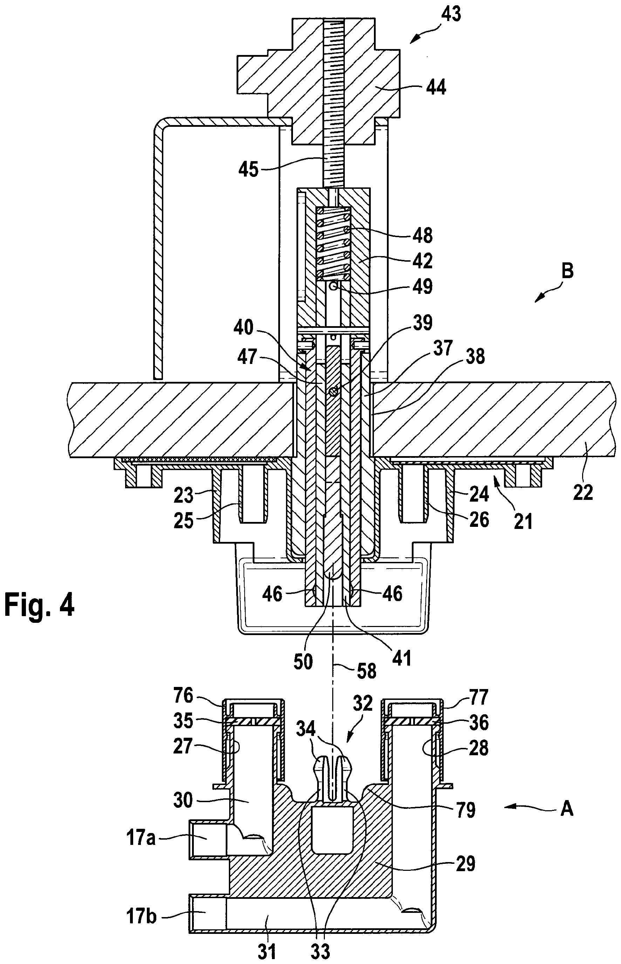

Fig. 4 zeigt die Position des Aufnahmestücks 40 mit dem Tastelement 47, in der die Spindel 45 des Linearmotors 44 ausgefahren ist und das Aufnahmestück 40 mit dem Tastelement 47 aus dem Führungsstück 37 nach außen vorgeschoben ist. In dieser Position ist der stiftförmige Körper 50, der mit dem Führungsstück 37 verbunden ist, soweit zurückgezogen, dass sich das Ansatzstück 32 der Stecker-Einheit A in das Aufnahmestück 40 einsetzen lässt.

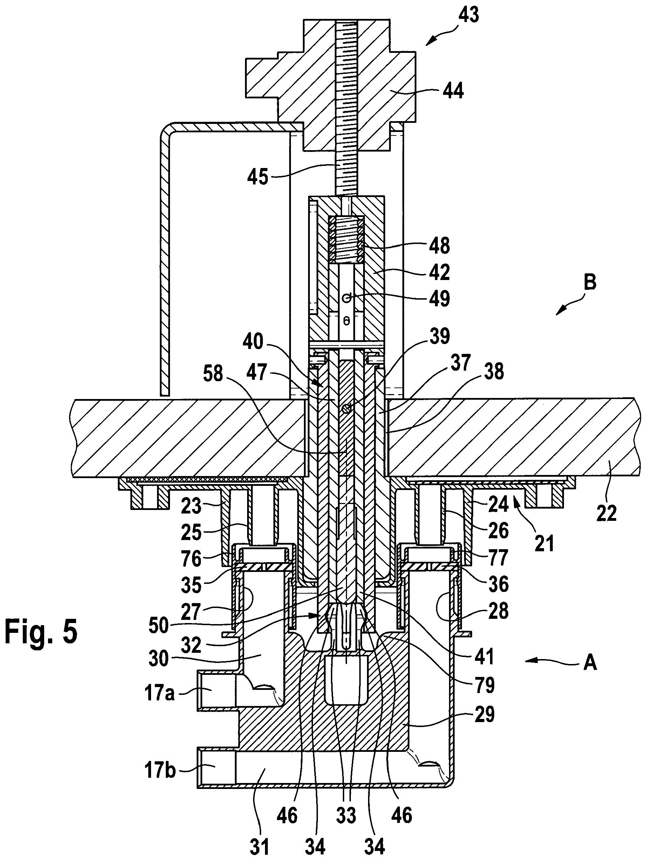

Fig. 5 zeigt das in dem Aufnahmestück 40 der Buchsen-Einheit B sitzende Ansatzstück 32 der Stecker-Einheit A. Beim Einsetzen des Ansatzstücks 32 in das Aufnahmestück 40 rasten die Rastnasen 34 der Rastelemente 33 einschnappend in die Ausnehmungen 46 des Aufnahmestücks 40 ein. Dadurch wird die Stecker-Einheit A lose an der Buchsen-Einheit gehalten. Die Strömungsverbindung ist dabei aber noch nicht hergestellt, da die Anschlussstücke 25, 26 der Buchsen-Einheit B noch nicht mit den Konnektoren 27, 28 der Steckereinheit A verbunden sind. Die Zulauf- und Ablaufkanäle 30, 31 der Stecker-Einheit werden in dieser Position noch von den Membranen 35, 36 flüssigkeitsdicht verschlossen.

Beim Einsetzen des Ansatzstücks 32 in das Aufnahmestück 40 wird das in dem Aufnahmestück längsverschiebbar geführte Tastelement 47 von den Rastelementen 33 des Ansatzstücks 32 entgegen der Federkraft der Feder 48 in das Aufnahmestück 40 zurückgeschoben. Die rückwärtige Stellung des Tastelements 47, die in Fig. 5 dargestellt ist, wird von nicht dargestellten Mitteln, beispielsweise elektrischen Kontakten, die geschlossen werden, oder einer Lichtschranke, erfasst, wodurch die Antriebseinheit 43 in Betrieb gesetzt wird. Der Linearmotor 44 fährt nun die Spindel 45 ein, sodass das Aufnahmestück 40 mit dem Tastelement 47 zurückgezogen wird.

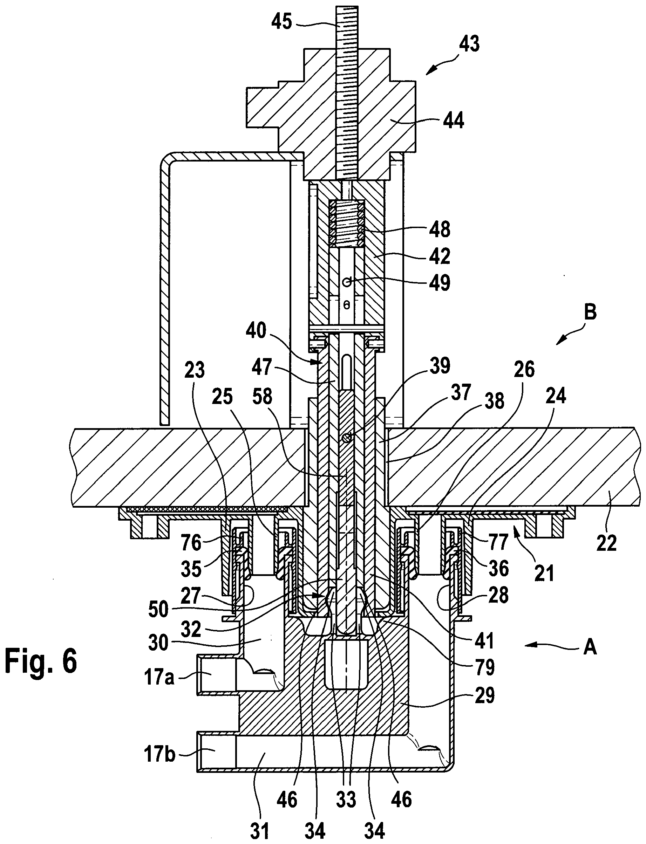

Fig. 6 zeigt die Position des Aufnahmestücks 40, wenn die Spindel 45 des Linearmotors 44 vollständig eingefahren und das Aufnahmestück 40 mit dem Tastelement 37 vollständig zurückgezogen ist. Beim Zurückziehen des Aufnahmestücks 40 wird der stiftförmige Körper 50 in das Ansatzstück 32 vorgeschoben, wodurch die Rastnasen 34 der Rastelemente 33 des Ansatzstücks 32 in den Ausnehmungen 46 des Aufnahmestücks 40 gesichert werden. Dadurch ist das Ansatzstück 32 in dem Aufnahmestück 40 verriegelt. Die Verriegelung des Ansatzstücks 32 in dem Aufnahmestück 40 erfolgt gleichzeitig mit der Relativbewegung der Anschlussstücke 25, 26 und der Konnektoren 27, 28.

In der in Fig. 6 dargestellten Position, in der die Spindel 45 des Linearmotors 44 vollständig eingefahren ist, sind beide Anschlussstücke 25, 26 und Konnektoren 27, 28 flüssigkeitsdicht miteinander verbunden. Die Verriegelung des Ansatzstücks 32 im Aufnahmestück 40 stellt zum einen sicher, dass die zunächst nur lose in die Buchsen-Einheit B eingesetzte Stecker-Einheit A entgegen den auftretenden Kräften auf die Buchsen-Einheit gezogen werden kann und verhindert zum anderen, dass nach Herstellung der Strömungsverbindungen sich die Stecker-Einheit von der Buchsen-Einheit lösen kann. Die flüssigkeitsdichte und nicht lösbare Verbindung zwischen Stecker-Einheit A und Buchsen-Einheit B wird also automatisch nach dem losen Einsetzten der Stecker-Einheit in die Buchsen-Einheit hergestellt.

Die Entriegelung der Stecker-Einheit A von der Buchsen-Einheit B erfolgt in umgekehrter Reihenfolge wie die Verriegelung der Stecker-Einheit an der Buchsen-Einheit. Hierzu wird die Antriebseinheit 43 wieder in Betrieb gesetzt. Dies kann beispielsweise durch Drücken eines Tasters oder dergleichen erfolgen. Wenn die Spindel 45 des Linearmotors 44 wieder ausgefahren wird, schiebt sich das Aufnahmestück 40 mit dem Tastelement 47 wieder über den stiftförmigen Körper 50 nach vorne, wodurch die Verriegelung der Rastverbindung zwischen Ansatzstück 32 und Aufnahmestück 40 aufgehoben wird. Gleichzeitig werden die Anschlussstücke 25, 26 von den Konnektoren 27, 28 getrennt. Damit befindet sich die Stecker-Einheit A wieder in der Ausgangsposition (Fig. 5), in der die Stecker-Einheit noch lose an der Buchsen-Einheit gehalten wird. Dadurch wird verhindert, dass die Stecker-Einheit von der Buchsen-Einheit einfach abfällt.

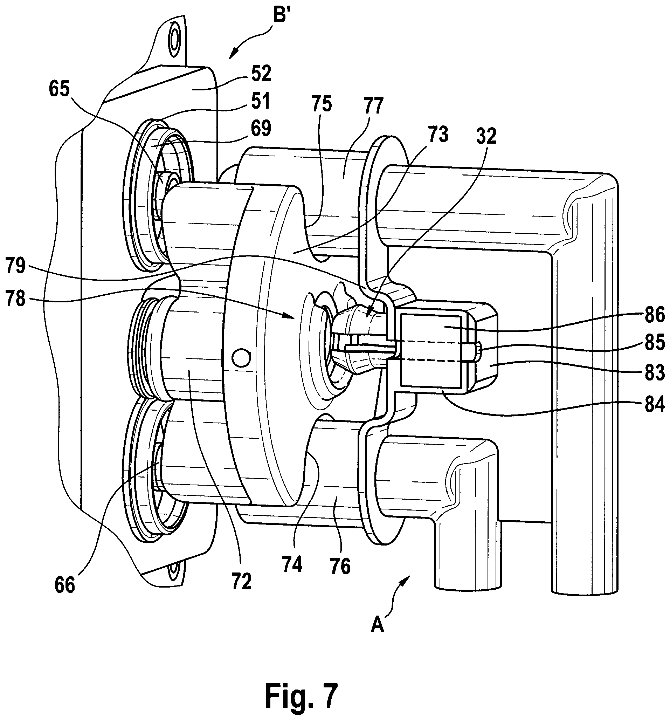

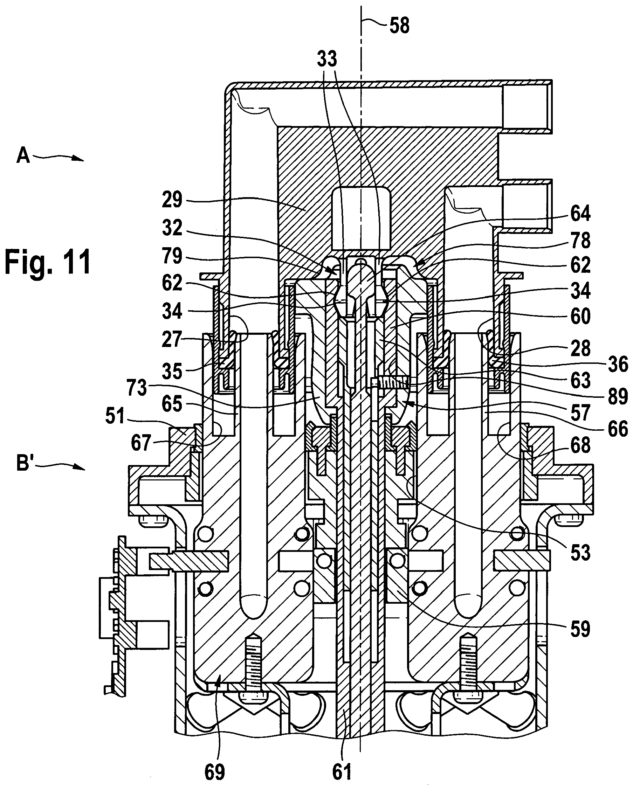

Die Figuren 7-11 zeigen eine alternative Ausführungsform der Buchsen-Einheit B', die an der Vorrichtung 3 zur Befüllung der Vorrichtung 1 zur Bereitstellung der Dialysierflüssigkeit vorgesehen ist. Diese alternative Ausführungsform kann grundsätzlich auch bei der Blutbehandlungsvorrichtung 2 vorgesehen sein. Es ist aber auch möglich, dass die unter Bezugnahme auf die Figuren 2 bis 6 beschriebene Ausführungsform bei der Vorrichtung 3 zur Befüllung vorgesehen ist. Die alternative Ausführungsform der Buchsen-Einheit B' wird nachfolgend im Einzelnen beschrieben.

Fig. 7 zeigt die alternative Ausführungsform der Buchsen-Einheit B' zusammen mit der Stecker-Einheit A in perspektivischer Darstellung. Beide Ausführungsformen der Buchsen-Einheit B und B' können mit derselben Stecker-Einheit A konnektiert werden, sodass sich die Vorrichtung 1 zur Bereitstellung von Dialysierflüssigkeit einerseits an die Vorrichtung 3 zum Befüllen und Entleeren und andererseits an die Blutbehandlungsvorrichtung 2 anschließen lässt.

Die beiden Ausführungsformen der Buchsen-Einheit unterscheiden sich insbesondere dadurch voneinander, dass beim Anschließen der Stecker-Einheit A an die Buchsen-Einheit B zur Herstellung der Strömungsverbindungen die Konnektoren 27, 28 der Stecker-Einheit A automatisch auf die Anschlussstücke 25, 26 der Buchsen-Einheit B gezogen werden (Figuren 2 bis 6), in dem die Stecker-Einheit A bewegt wird, während bei der alternativen Ausführungsform der Buchseneinheit B' die Anschlussstücke der Buchsen-Einheit in die Konnektoren 27, 28 der Stecker-Einheit A eingefahren werden, wobei die Stecker-Einheit A nicht bewegt wird. Darüber hinaus sieht die alternative Ausführungsform der Buchsen-Einheit B' den Verschluss der beiden Anschlussstücke oder die Herstellung einer Strömungsverbindung zwischen den beiden Anschlussstücken für einen Spülvorgang vor, ohne dass die Stecker-Einheit A und Buchsen-Einheit B' miteinander verbunden sind.

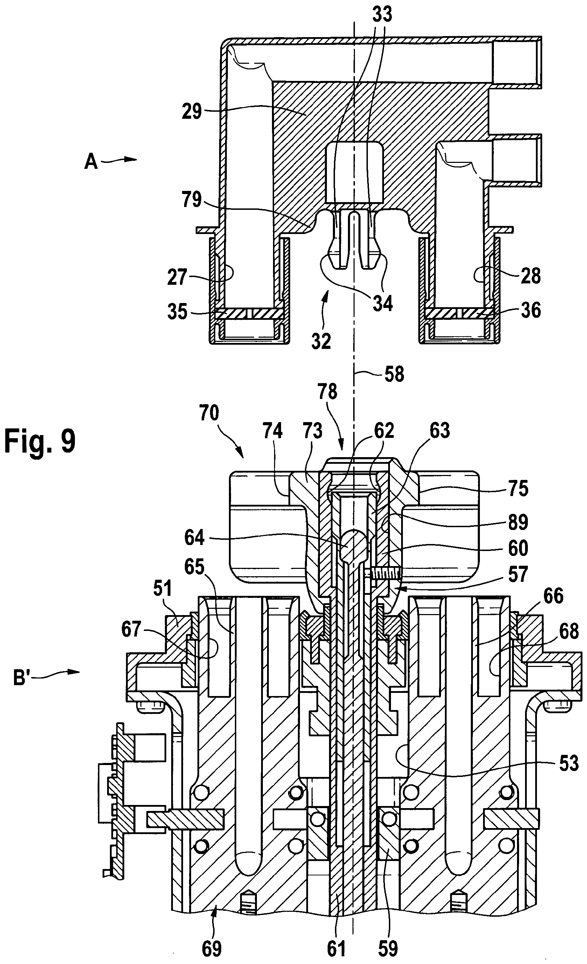

Die Buchsen-Einheit B' weist einen Gehäusekörper 51 auf, der in einer Gehäusewand 52 der Vorrichtung 3 zum Befüllen eingesetzt ist (Fig. 7). Der Gehäusekörper 51 weist eine zentrale Ausnehmung 53 auf, in der das Aufnahmestück 57 für das Ansatzstück 32 der Stecker-Einheit A angeordnet ist (Fig. 9). Im Gegensatz zu der unter Bezugnahme auf die Figuren 2-6 beschriebenen Ausführungsform ist das Aufnahmestück 57 der Buchsen-Einheit B' nicht in Richtung der Längsachse 58 der Buchsen-Einheit B' verschiebbar geführt, sondern um die Längsachse 58 mit einem Lager 59 drehbar gelagert, das in die zentrale Ausnehmung 53 des Gehäusekörpers 51 eingesetzt ist. Das Aufnahmestück 57 wird mit einer nicht dargestellten Antriebseinheit gedreht.

Das Aufnahmestück 57 weist einen sich aus dem Gehäusekörper 51 erstreckenden vorderen Abschnitt 60 und einen sich in den Gehäusekörper 51 erstreckenden hinteren Abschnitt 61 auf, wobei der vordere Abschnitt 60 einen größeren Außen- bzw. Innendurchmesser als der hintere Abschnitt 61 hat. An der Innenseite des vorderen Endes des vorderen Abschnitts 60 des Aufnahmestücks 57 sind die umfangsgemäß verteilt angeordneten Ausnehmungen 62 vorgesehen, in die die Rastnasen 34 der Rastelemente 33 des Ansatzstücks 32 einrasten, wenn die Stecker-Einheit A lose auf die Buchsen-Einheit B' aufgesetzt wird.

In dem rohrförmigen Aufnahmestück 57 ist längsverschiebbar das als rohrförmiger Körper ausgebildete Tastelement 63 geführt, dass mit einer nicht dargestellten Feder vorgespannt ist, sodass beim Einsetzen des Ansatzstücks 32 in das Aufnahmestück 57 das Tastelement 63 entgegen der Federspannung zurückgeschoben wird.

In dem rohrförmigen Tastelement 63 ist der stiftförmige Körper 64 zum Verriegeln des Ansatzstücks 32 in dem Aufnahmestück 57 geführt. Der stiftförmige Körper 64 kann mit einer nicht dargestellten Antriebseinheit in Längsrichtung der Achse 58 vorgeschoben und wieder zurückgezogen werden, um das Ansatzstück 32 in dem Aufnahmestück 57 freizugeben bzw. zu verriegeln.

Bei der alternativen Ausführungsform der Buchseneinheit B' sitzen die Anschlussstücke 65, 66 in zylindrischen Ausnehmungen 67, 68 eines Anschlussteils 69, das in dem Gehäusekörper 51 längsverschiebbar geführt ist, sodass die Anschlussstücke 65, 66 aus dem Gehäusekörper 51 vorgeschoben bzw. in den Gehäusekörper zurückgezogen werden können. Die Antriebseinheit zum Vorschieben bzw. Zurückziehen des Anschlussteils 69 mit den Anschlussstücken 65, 66 ist in den Figuren nicht dargestellt.

Fig. 9 zeigt die Buchsen-Einheit B' in der Position, in der die Stecker-Einheit A lose auf die Buchsen-Einheit B' aufgesetzt werden kann. Der stiftförmige Körper 64 ist in dem Aufnahmestück 57 zurückgezogen, sodass die Rastelemente 33 mit den Rastnasen 34 des Ansatzstücks 32 in das Aufnahmestück 57 mit den Ausnehmungen 62 einrasten kann.

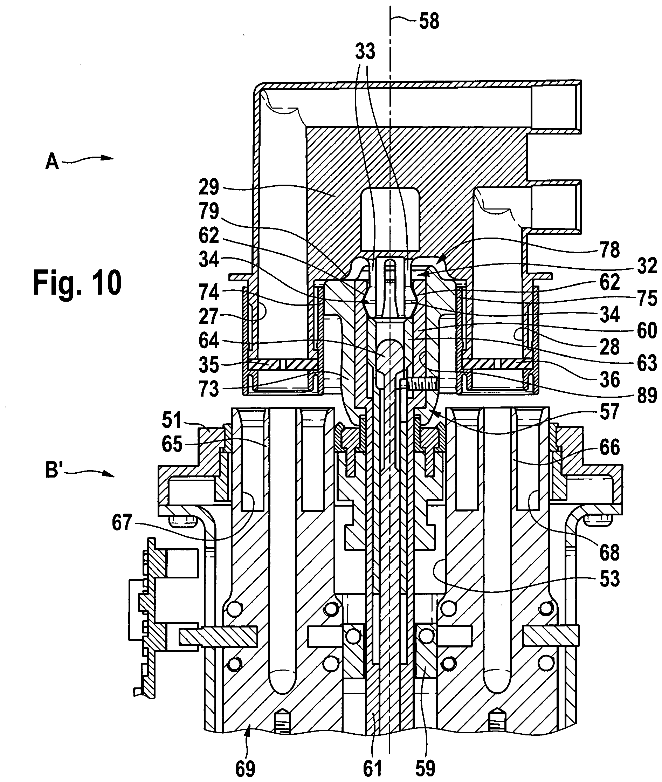

Fig. 10 zeigt die Position, in der die Stecker-Einheit A lose auf die Buchsen-Einheit B' aufgesetzt ist, wobei das Ansatzstück 32 in das Aufnahmestück 57 eingerastet ist. Die Stecker-Einheit A wird dabei nur lose gehalten, ohne dass die Strömungsverbindungen hergestellt sind.

Die Stellung des Tastelements 63 wird wieder überwacht. Da das Tastelement 63 von dem Ansatzstück 32 zurückgeschoben ist, wird erkannt, dass die Stecker-Einheit A lose aufgesetzt ist. Wenn die Stecker-Einheit lose aufgesetzt ist, wird die nicht dargestellte Antriebseinheit in Betrieb gesetzt, wodurch der stiftförmige Körper 64 in dem Aufnahmestück 57 nach vorne geschoben wird. Dadurch wird die zunächst erst lose Verbindung zwischen Ansatzstück 32 und Aufnahmestück 57 verriegelt. Gleichzeitig wird der Anschlussteil 69 mit den beiden Anschlussstücken 65, 66 aus dem Gehäusekörper 51 nach vorne geschoben. Es ist auch möglich, dass der stiftförmige Körper 64 und der Anschlussteil 69 miteinander verbunden sind und von einer Antriebseinheit gemeinsam bewegt werden. Mit dem Verschieben des Anschlussteils 69 mit den Anschlussstücken 65, 66 durchstoßen die Anschlussstücke 65, 66 die Membranen 35, 36 der Stecker-Einheit A, wodurch die flüssigkeitsdichten Verbindungen zwischen den Anschlussstücken und Konnektoren hergestellt wird. Zwar findet wieder eine Relativbewegung zwischen Anschlussstücken 65, 66 und Konnektoren 27, 28 statt. Bei dieser Ausführungsform wird die Stecker-Einheit A aber selbst nicht bewegt. Da die Stecker-Einheit A nach der Verriegelung des Ansatzstücks mit dem Aufnahmestück fest auf der Buchsen-Einheit B' sitzt, können die beim Verbinden von Stecker- und Buchsen-Einheit auftretenden Kräfte aufgenommen werden. Der Anschluss der Stecker-Einheit an die Buchsen-Einheit erfolgt also wieder automatisch.

Das Lösen der Stecker-Einheit A von der Buchsen-Einheit B' erfolgt in umgekehrter Reihenfolge. Hierzu werden der stiftförmige Körper 64 in dem Aufnahmestück 57 und das Anschlussteil 69 mit den Anschlussstücken 65, 66 in dem Gehäusekörper 51 zurückgezogen, wodurch die Verbindung zwischen Ansatzstück 32 und Aufnahmestück 57 entriegelt und die Anschlussstücke 65, 66 aus den Konnektoren 27, 28 gezogen werden. Die Entriegelung kann gleichzeitig mit dem Zurückziehen der Anschlussstücke oder vor dem Zurückziehen der Anschlussstücke erfolgen.

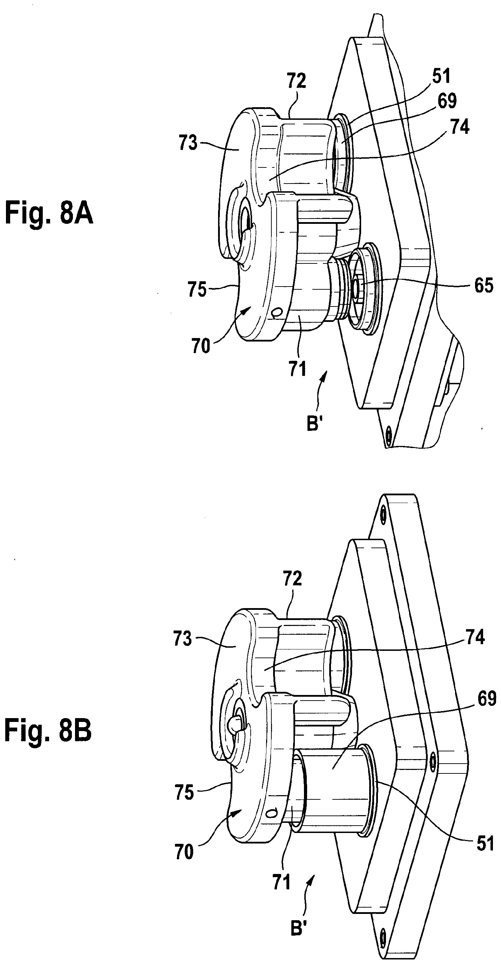

Die alternative Ausführungsform der Buchsen-Einheit B' verfügt über eine Einrichtung 70 zum Verschluss der beiden Anschlussstücke 65, 66 oder der Herstellung einer Strömungsverbindung zwischen den beiden Anschlussstücken 65, 66, um einen Spülvorgang mit einer Spüllösung durchführen zu können (Fig. 7; Fig. 8A und Fig. 8B). Diese Einrichtung 70 weist zwei Konnektoren 71, 72 auf, die im gleichen Abstand wie die Konnektoren 27, 28 der Stecker-Einheit A zueinander angeordnet sind und die gleiche Ausbildung aufweisen wie die Konnektoren der Stecker-Einheit. Die beiden Konnektoren 71, 72 sind in einem Spülstück 73 an ihrem rückwärtigen Ende verschlossen oder sind in dem Spülstück 73 zur Herstellung einer Strömungsverbindung verbunden.

Das Spülstück 73 weist an den beiden gegenüberliegenden Seiten, an denen die Konnektoren 71, 72 nicht angeordnet sind, halbkreisförmige Einschnitte 74, 75 auf. Das Spülstück 73 mit den Konnektoren 71, 72 ist mit dem vorderen Abschnitt 60 des Aufnahmestücks 57 der Buchsen-Einheit B' verbunden. Dazu weist das Spülstück 73 eine zentrale Ausnehmung 89 auf, durch die sich der vordere Abschnitt 60 des Aufnahmestücks 57 erstreckt (Fig. 9). Da das Aufnahmestück 57 um die Längsachse 58 drehbar gelagert ist, kann durch Drehen des Aufnahmestücks 57 mit der nicht dargestellten Antriebseinheit auch das Spülstück 73 mit den Konnektoren 71, 72 um die Längsachse 58 gedreht werden.

Fig. 7 zeigt das Spülstück 73 mit den Konnektoren 71, 72 in der Position, in der sich die Stecker-Einheit A auf die Buchsen-Einheit B' aufsetzen lässt. In dieser Position befinden sich die halbkreisförmigen Ausnehmungen 74, 75 vor den Anschlussstücken 65, 66 der Buchsen-Einheit B', während die Konnektoren 71, 72 in einer Ebene angeordnet sind, die senkrecht auf der Ebene steht, in der die Anschlussstücke 65, 66 angeordnet sind.

Für die Einleitung des Spülvorgangs wird das Spülstück 73 mit den Konnektoren 71, 72 durch Drehen des Aufnahmestücks 57 von der nicht dargestellten Antriebseinheit um 90° verschwenkt, sodass sich die Konnektoren 71, 72 vor den Anschlusstücken 65, 66 befinden. Damit sind die Anschlussstücke aber noch nicht verschlossen oder eine Strömungsverbindung zwischen den Anschlussstücken ist aber noch nicht hergestellt (Fig. 8A). Daraufhin wird der Anschlussteil 69 mit den Anschlussstücken 65, 66 aus dem Gehäusekörper 51 vorgeschoben, sodass die Konnektoren 65, 66 in die Membranen der Konnektoren 71, 72 eingeschoben werden. Damit wird eine flüssigkeitsdichte Strömungsverbindung zwischen den Anschlussstücken 65, 66 der Buchsen-Einheit B' und den Konnektoren 71, 72 hergestellt, sodass die beiden Anschlussstücke 65, 66 über das Spülstück 73 verschlossen oder kurzgeschlossen sind (Fig. 8B). Nach Beendigung des Spülvorgangs werden die Anschlussstücke 65, 66 wieder zurückgezogen und das Spülstück 73 mit den Konnektoren 71, 72 in die Ausgangsposition (Fig.7) zurückgedreht.

Das Spülstück 73 der Buchsen-Einheit (B') und der Steckerkörper 29 der Stecker-Einheit A sind asymmetrisch ausgebildet, so dass sich die Stecker-Einheit A nur in der in Fig. 7 gezeigten korrekten Stellung in die Buchsen-Einheit (B') einstecken lässt. Hierzu weist das Spülstück 73 an der dem Steckerkörper gegenüberliegenden Außenseite einen U-förmigen Ansatz 78 auf, während der Steckerkörper 29 an der dem Spülstück gegenüberliegenden Außenseite einen Vorsprung 79 aufweist. Der Vorsprung 79 ist derart angeordnet, dass er gegen den Ansatz 78 stößt, wenn die Stecker-Einheit A in einer um 180° gedrehten Stellung in die Buchsen-Einheit (B') eingesteckt wird.

Die besondere Ausbildung der Einrichtung 70 zum Verschluss oder zur Herstellung der Strömungsverbindung stellt einen Bestandteil der Buchsen-Einheit B' dar. Ein separater Stecker oder dergleichen ist daher nicht erforderlich. Die Buchsen-Einheit B' erlaubt eine vollautomatische Steuerung sowohl des Anschlusses der Stecker-Einheit A an die Buchsen-Einheit B' als auch der Einleitung des Spülvorgangs, sodass die Handhabung insgesamt vereinfacht wird. Da das Einsetzen der Stecker-Einheit in die Buchsen-Einheit erkannt wird, kann der Befüllvorgang oder Entleervorgang automatisch eingeleitet werden. Der Befüllvorgang kann aber auch verhindert werden, wenn der Informationsträger 86 an der Stecker-Einheit A zerstört ist. Vorzugsweise verhindert die Steuereinheit 80 der Vorrichtung 3 zum Befüllen, dass die Anschlussstücke 65, 66 der Buchsen-Einheit B zur Herstellung einer Strömungsverbindung ausgefahren werden. Nach dem Befüllen oder Entleeren kann die Stecker-Einheit automatisch freigegeben werden. Das gleiche gilt auch für den Spülvorgang. Auch beim Anschluss der Vorrichtung 1 zur Bereitstellung von Dialysierflüssigkeit an die Dialysevorrichtung 2 kann die Befüllung des Flüssigkeitsreservoirs 10 mit dem Einsetzen der Stecker-Einheit A in die Buchsen-Einheit B der Dialysevorrichtung automatisch gestartet werden.

## Patentansprüche

1. Medizinisches Behandlungssystem mit
**mindestens einer Vorrichtung (1) zur Bereitstellung einer medizinischen Flüssigkeit für eine extrakorporale Dialysevorrichtung oder eine Vorrichtung zur Peritonealdialyse,** mit
einer Stecker-Einheit (A) zum Anschluss an eine Buchsen-Einheit der Dialysevorrichtung oder Vorrichtung zur Peritonealdialyse (2), wobei die Stecker-Einheit (A) Mittel (86) zur Signalisierung von zwei Betriebszuständen aufweist, die derart ausgebildet sind, dass die Mittel (86) zur Signalisierung von zwei Betriebszuständen beim Anschluss der Stecker-Einheit (A) an die Buchsen-Einheit der Dialysevorrichtung oder Vorrichtung zur Peritonealdialyse (2) beschädigbar oder zerstörbar sind,
wobei der erste Betriebszustand der Zustand vor dem Anschluss der Stecker-Einheit (A) an die Buchsen-Einheit (B), wenn die Mittel unversehrt sind, und der zweite Betriebszustand der Zustand nach dem Anschluss der Stecker-Einheit (A) an die Buchsen-Einheit (B) ist, wenn die Mittel beschädigt oder zerstört sind,
**mindestens einer extrakorporalen Dialysevorrichtung oder Vorrichtung zur Peritonealdialyse (2),** mit
einer Buchsen-Einheit (B) zum Anschluss der Stecker-Einheit der Vorrichtung (1) zur Bereitstellung einer medizinischen Flüssigkeit für die Dialysevorrichtung oder Vorrichtung zur Peritonealdialyse (2), wobei die Buchsen-Einheit (B) Mittel (88) zum Beschädigen oder Zerstören der Mittel zur Signalisierung von zwei Betriebszuständen der Vorrichtung (1) zur Bereitstellung von einer medizinischen Flüssigkeit aufweist, die derart ausgebildet sind, dass die Mittel zur Signalisierung von zwei Betriebszuständen beim Anschluss der Stecker-Einheit der Vorrichtung zur Bereitstellung einer medizinischen Flüssigkeit an die Buchsen-Einheit (B) beschädigt oder zerstört werden,
wobei der erste Betriebszustand der Zustand vor dem Anschluss der Stecker-Einheit (A) an die Buchsen-Einheit (B), wenn die Mittel unversehrt sind, und der zweite Betriebszustand der Zustand nach dem Anschluss der Stecker-Einheit (A) an die Buchsen-Einheit (B) ist, wenn die Mittel beschädigt oder zerstört sind,
**mindestens einer Vorrichtung (3) zur Befüllung der Vorrichtung zur Bereitstellung von einer medizinischen Flüssigkeit,** mit
einer Buchsen-Einheit (B') zum Anschluss der Stecker-Einheit (A) der Vorrichtung (1) zur Bereitstellung einer medizinischen Flüssigkeit, wobei die Vorrichtung zur Befüllung (3) Mittel (81; 82) zur Erkennung der Wiederverwendung der Vorrichtung zur Bereitstellung einer medizinischen Flüssigkeit aufweist, die derart ausgebildet sind, dass detektiert wird, ob die Mittel zur Signalisierung von zwei Betriebszuständen der Stecker-Einheit (A) der Vorrichtung (1) zur Bereitstellung einer medizinischen Flüssigkeit unversehrt bzw. beschädigt oder zerstört sind.

2. Medizinisches Behandlungssystem nach Anspruch 1, **dadurch gekennzeichnet, dass** die Mittel (88) zum Beschädigen oder Zerstören einen an einem Teilstück der Buchsen-Einheit (B) der Dialysevorrichtung oder Vorrichtung zur Peritonealdialyse (2) vorgesehenen vorspringenden Ansatz (88) aufweisen, der beim Anschluss der Stecker-Einheit (A) der Vorrichtung (1) zur Bereitstellung einer medizinischen Flüssigkeit an die Buchsen-Einheit (B) der Dialysevorrichtung oder Vorrichtung zur Peritonealdialyse (2) in eine Vertiefung eines Teilstücks der Stecker-Einheit (A) greifen kann, auf der ein flächiger Informationsträger aufgebracht ist.

3. Medizinisches Behandlungssystem nach Anspruch 2, **dadurch gekennzeichnet, dass** die Mittel (81; 82) zur Erkennung der Wiederverwendung der Vorrichtung (3) zum Befüllen eine Einheit (81) zum Lesen von Informationen auf dem flächigen Informationsträger aufweisen, und die Mittel (81; 82) zur Erkennung der Wiederverwendung eine Auswerteinheit (82) aufweisen, die derart ausgebildet ist, dass ein die Wiederverwendung signalisierendes Signal erzeugt wird, wenn nach der Beschädigung oder Zerstörung des Informationsträgers die Auslesung von Informationen fehlerhaft ist.

4. Medizinisches Behandlungssystem nach Anspruch 3, **dadurch gekennzeichnet, dass** die Vorrichtung (3) zur Befüllung eine Steuereinheit (80) zur Steuerung des Befüllvorgangs aufweist, die derart ausgebildet ist, dass die Einleitung des Befüllvorgangs verhindert wird, wenn von der Auswerteinheit (82) das die Wiederverwendung signalisierende Signal erzeugt wird.

5. Medizinisches Behandlungssystem nach Anspruch 3 oder 4, **dadurch gekennzeichnet, dass** die Einheit zum Lesen von Informationen ein Lesegerät (81) für einen Matrixcode ist.

6. Medizinisches Behandlungssystem nach einem der Ansprüche 2 bis 5, **dadurch gekennzeichnet, dass** die Mittel zur Signalisierung von zwei Betriebszuständen einen flächigen Informationsträger (86) aufweisen, der maschinenlesbare Informationen über charakteristische Eigenschaften der medizinischen Flüssigkeit trägt.

7. Medizinisches Behandlungssystem nach einem der Ansprüche 2 bis 6, **dadurch gekennzeichnet, dass** der Informationsträger (86) auf einem Teilstück (83) der Stecker-Einheit (A) der Vorrichtung (1) zur Bereitstellung einer medizinischen Flüssigkeit aufgebracht ist, der mit einer Vertiefung (85) versehen ist, so dass der Informationsträger durch einen in die Vertiefung greifenden vorspringenden Ansatz der Buchsen-Einheit (B) der Dialysevorrichtung oder Vorrichtung zur Peritonealdialyse (2) beim Anschluss der Stecker-Einheit (A) an die Buchsen-Einheit (B) beschädigt oder zerstört wird.

8. Medizinisches Behandlungssystem nach Anspruch 7, **dadurch gekennzeichnet, dass** die Vertiefung eine zumindest an einem Ende offene Nut (85) in dem Teilstück (83) der Stecker-Einheit (A) der Vorrichtung (1) zur Bereitstellung einer medizinischen Flüssigkeit ist, die parallel zu der Achse (58) der Stecker-Einheit (A) verläuft, in deren Richtung die Stecker-Einheit (A) in die Buchsen-Einheit (B) der Dialysevorrichtung oder Vorrichtung zur Peritonealdialyse (2) eingesteckt wird, so dass beim Einstecken der Stecker-Einheit (A) in die Buchsen-Einheit der vorspringende Ansatz in die Nut (85) eingeschoben wird.

9. Medizinisches Behandlungssystem nach einem der Ansprüche 1 bis 8, **dadurch gekennzeichnet, dass** die Stecker-Einheit (A) der Vorrichtung (1) zur Bereitstellung einer medizinischen Flüssigkeit zwei Konnektoren (27, 28) zum Anschluss von zwei Anschlussstücken der Buchsen-Einheit (B) der Dialysevorrichtung oder Vorrichtung zur Peritonealdialyse (2) aufweist, so dass eine Strömungsverbindung zum Zuführen frischer und Abführen verbrauchter Flüssigkeit herstellbar ist.

10. Medizinisches Behandlungssystem nach einem der Ansprüche 1 bis 9, **dadurch gekennzeichnet, dass** die Buchsen-Einheit (B) der Dialysevorrichtung oder Vorrichtung zur Peritonealdialyse (2) zwei Anschlussstücke (25, 26) zum Anschluss von zwei Konnektoren der Stecker-Einheit (A) der Vorrichtung (1) zur Bereitstellung einer medizinischen Flüssigkeit aufweist, so dass eine Strömungsverbindung zum Zuführen frischer oder Abführen verbrauchter Flüssigkeit herstellbar ist.

## Claims

1. Medical treatment system having
at least one device (1) for supplying a medical fluid for an extra-corporeal dialysis apparatus or an apparatus for peritoneal dialysis, having
a plug unit (A) for connection to a socket unit of the extra-corporeal dialysis apparatus or apparatus for peritoneal dialysis (2), the plug unit (A) having means (86) for indicating two states of operation which are so designed that the means (86) for indicating two states of operation can be damaged or destroyed when the plug unit (A) is connected to the socket unit of the extra-corporeal dialysis apparatus or apparatus for peritoneal dialysis,
the first state of operation being the state before the plug unit (A) is connected to the socket unit (B), when the means are intact, and the second state of operation being the state after the plug unit (A) has been connected to the socket unit (B), when the means are damaged or destroyed
at least one extra-corporeal dialysis apparatus or apparatus for peritoneal dialysis (2), having
a socket unit (B) for the connection of the plug unit of the device (1) for supplying a medical fluid for the extra-corporeal dialysis apparatus or apparatus for peritoneal dialysis (2), the socket unit (B) having means (88) for damaging or destroying the means for indicating two states of operation of the device (1) for supplying a medical fluid, which means are so designed that the means for indicating two states of operation are damaged or destroyed when the plug unit of the device for supplying a medical fluid is connected to the socket unit (B),
the first state of operation being the state before the plug unit (A) is connected to the socket unit (B), when the means are intact, and the second state of operation being the state after the plug unit (A) has been connected to the socket unit (B), when the means are damaged or destroyed,
at least one apparatus (3) for filling the device for supplying a medical fluid, having
a socket unit (B') for the connection of the plug unit (A) of the device (1) for supplying a medical fluid, the filling apparatus (3) having means (81; 82) for detecting re-use of the device for supplying a medical fluid which are so designed that it is detected whether the means for indicating two states of operation of the plug unit (A) of the device (1) for supplying a medical fluid are intact or are damaged or destroyed.

2. Medical treatment system according to claim 1, **characterised in that** the means (88) for damaging or destroying have a salient projection (88) which is provided on a portion of the socket unit (B) of the of the extra-corporeal dialysis apparatus or apparatus for peritoneal dialysis (2) and which, when the plug unit (A) of the device (1) for supplying a medical fluid is connected to the socket unit (B) of the extra-corporeal dialysis apparatus or apparatus for peritoneal dialysis (2), is able to engage in a depression in a portion of the plug unit (A) to which a planar information carrier is applied.

3. Medical treatment system according to claim 2, **characterised in that** the means (81; 82) for detecting re-use of the filling apparatus (3) have a unit (81) for reading information on a planar information carrier, and the means (81, 82) for detecting re-use have an analysing unit (82) which is so designed that a signal indicating re-use is generated when the read-out of information is faulty after the damaging or destruction of the information carrier.

4. Medical treatment system according to claim 3, **characterised in that** the filling apparatus (3) has a control unit (80) for controlling the filling process which is so designed that the initiation of the filling process is prevented if the analysing unit (82) generates the signal indicating re-use.

5. Medical treatment system according to claim 3 or 4, **characterised in that** the unit for reading information is a reader (81) for reading a matrix code.

6. Medical treatment system according to one of claims 2 to 5, **characterised in that** the means for indicating two states of operation have a planar information carrier (86) which carries machine-readable information on characteristic properties of the medical fluid.

7. Medical treatment system according to one of claims 2 to 6, **characterised in that** that the information carrier (86) is applied to a portion (83) of the plug unit (A) of the device (1) for supplying a medical fluid, which is provided with a depression (85), the information carrier thus being damaged or destroyed, when the plug unit (A) is connected to the socket unit (B), by a projection on the socket unit (B) of the dialysis apparatus or apparatus for peritoneal dialysis (2), which engages in the depression.

8. Medical treatment system according to claim 7, **characterised in that** the depression is a groove (85) in the portion (83) of the plug unit (A) of the device (1) for supplying a medical fluid, which groove is open at at least one end and extends parallel to the axis (58) of the plug unit (A), which axis defines the direction in which the plug unit (A) is plugged into the socket unit (B) of the dialysis apparatus or apparatus for peritoneal dialysis (2), the salient projection thus being thrust into the groove (85) when the plug unit (A) is plugged into the socket unit.

9. Medical treatment system according to one of claims 1 to 8, **characterised in that** the plug unit (A) of the device (1) for supplying a medical fluid has two connectors (27, 28) for the connection of two connecting pieces of the socket unit (B) of the dialysis apparatus or apparatus for peritoneal dialysis (2), thus enabling a flow-permitting connection to be made for feeding in fresh fluid and feeding out used fluid.

10. Medical treatment system according to one of claims 1 to 9, **characterised in that** the socket unit (B) of the dialysis apparatus or apparatus for peritoneal dialysis (2) has two connecting pieces (25, 26) for the connection of two connectors of the plug unit (A) of the device (1) for supplying a medical fluid, thus enabling a flow-permitting connection to be made to feed fresh fluid in or to feed used fluid out.

## Revendications

1. Système de traitement médical avec
au moins un dispositif (1) de fourniture d'un liquide médical pour un dispositif de dialyse extracorporel ou un dispositif de dialyse péritonéale, avec
une unité connecteur mâle (A) pour le raccordement sur une unité connecteur femelle du dispositif de dialyse ou dispositif de dialyse péritonéale (2), dans lequel l'unité connecteur mâle (A) présente des moyens (86) de signalisation de deux états de fonctionnement, qui sont réalisés de telle sorte que les moyens (86) de signalisation de deux états de fonctionnement puissent être endommagés ou détruits lors du raccordement de l'unité connecteur mâle (A) sur l'unité connecteur femelle du dispositif de dialyse ou dispositif de dialyse péritonéale (2),
dans lequel le premier état de fonctionnement est l'état avant le raccordement de l'unité connecteur mâle (A) sur l'unité connecteur femelle (B), lorsque les moyens sont intacts, et le deuxième état de fonctionnement est l'état après le raccordement de l'unité connecteur mâle (A) sur l'unité connecteur femelle (B), lorsque les moyens sont endommagés ou détruits,
au moins un dispositif de dialyse extracorporel ou dispositif de dialyse péritonéale (2), avec
une unité connecteur femelle (B) pour le raccordement de l'unité connecteur mâle du dispositif (1) de fourniture d'un liquide médical pour le dispositif de dialyse ou dispositif de dialyse péritonéale (2), dans lequel l'unité connecteur femelle (B) présente des moyens (88) d'endommagement ou de destruction des moyens de signalisation de deux états de fonctionnement du dispositif (1) de fourniture d'un liquide médical, qui sont réalisés de telle sorte que les moyens de signalisation de deux états de fonctionnement soient endommagés ou détruits lors du raccordement de l'unité connecteur mâle du dispositif de fourniture d'un liquide médical sur l'unité connecteur femelle (B),
dans lequel le premier état de fonctionnement est l'état avant le raccordement de l'unité connecteur mâle (A) sur l'unité connecteur femelle (B), lorsque les moyens sont intacts, et le deuxième état de fonctionnement est l'état après le raccordement de l'unité connecteur mâle (A) sur l'unité connecteur femelle (B), lorsque les moyens sont endommagés ou détruits,
au moins un dispositif (3) de remplissage du dispositif de fourniture d'un liquide médical, avec
une unité connecteur femelle (B') pour le raccordement de l'unité connecteur mâle (A) du dispositif (1) de fourniture d'un liquide médical, dans lequel le dispositif de remplissage (3) présente des moyens (81 ; 82) de détection de la réutilisation du dispositif de fourniture d'un liquide médical, qui sont réalisés de telle sorte qu'il est détecté si les moyens de signalisation de deux états de fonctionnement de l'unité connecteur mâle (A) du dispositif (1) de fourniture d'un liquide médical sont intacts, ou endommagés ou détruits.

2. Système de traitement médical selon la revendication 1, **caractérisé en ce que** les moyens (88) d'endommagement ou de destruction présentent un appendice (88) en saillie prévu au niveau d'une partie de l'unité connecteur femelle (B) du dispositif de dialyse ou dispositif de dialyse péritonéale (2), qui peut venir en prise lors du raccordement de l'unité connecteur mâle (A) du dispositif (1) de fourniture d'un liquide médical sur l'unité connecteur femelle (B) du dispositif de dialyse ou dispositif de dialyse péritonéale (2) dans un évidement d'une partie de l'unité connecteur mâle (A), sur laquelle un support d'information plat est appliqué.

3. Système de traitement médical selon la revendication 2, **caractérisé en ce que** les moyens (81 ; 82) de détection de la réutilisation du dispositif (3) de remplissage présentent une unité (81) de lecture d'informations sur le support d'information plat, et les moyens (81 ; 82) de détection de la réutilisation présentent une unité d'évaluation (82), qui est réalisée de telle sorte qu'un signal signalant la réutilisation est généré, lorsqu'après l'endommagement ou la destruction du support d'information, le relevé d'informations est incorrect.

4. Système de traitement médical selon la revendication 3, **caractérisé en ce que** le dispositif (3) de remplissage présente une unité de commande (80) pour la commande du procédé de remplissage, qui est réalisée de telle sorte que l'initiation du procédé de remplissage est empêchée, lorsque le signal signalant la réutilisation est généré par l'unité d'évaluation (82).

5. Système de traitement médical selon la revendication 3 ou 4, **caractérisé en ce que** l'unité de lecture d'informations est un appareil de lecture (81) pour un code matriciel.

6. Système de traitement médical selon l'une quelconque des revendications 2 à 5, **caractérisé en ce que** les moyens de signalisation de deux états de fonctionnement présentent un support d'information plat (86), qui porte des informations lisibles par machine relatives à des propriétés caractéristiques du liquide médical.

7. Système de traitement médical selon l'une quelconque des revendications 2 à 6, **caractérisé en ce que** le support d'information (86) est appliqué sur une partie (83) de l'unité connecteur mâle (A) du dispositif (1) de fourniture d'un liquide médical, qui est dotée d'un évidement (85), si bien que le support d'information est endommagé ou détruit par un appendice en saillie venant en prise dans l'évidement de l'unité connecteur femelle (B) du dispositif de dialyse ou dispositif de dialyse péritonéale (2) lors du raccordement de l'unité connecteur mâle (A) sur l'unité connecteur femelle (B).

8. Système de traitement médical selon la revendication 7, **caractérisé en ce que** l'évidement est une rainure (85) au moins ouverte à une extrémité dans la partie (83) de l'unité connecteur mâle (A) du dispositif (1) de fourniture d'un liquide médical, qui s'étend parallèlement à l'axe (58) de l'unité connecteur mâle (A), dans la direction dans laquelle l'unité connecteur mâle (A) est branchée dans l'unité connecteur femelle (B) du dispositif de dialyse ou dispositif de dialyse péritonéale (2), si bien que lors du branchement de l'unité connecteur mâle (A) dans l'unité connecteur femelle l'appendice en saillie est inséré dans la rainure (85).

9. Système de traitement médical selon l'une quelconque des revendications 1 à 8, **caractérisé en ce que** l'unité connecteur mâle (A) du dispositif (1) de fourniture d'un liquide médical présente deux connecteurs (27, 28) pour le raccordement de deux raccords de l'unité connecteur femelle (B) du dispositif de dialyse ou dispositif de dialyse péritonéale (2), si bien qu'une liaison fluidique pour l'amenée de liquide frais et l'évacuation de liquide usagé peut être établie.

10. Système de traitement médical selon l'une quelconque des revendications 1 à 9, **caractérisé en ce que** l'unité connecteur femelle (B) du dispositif de dialyse ou dispositif de dialyse péritonéale (2) présente deux raccords (25, 26) pour le raccordement de deux connecteurs de l'unité connecteur mâle (A) du dispositif (1) de fourniture d'un liquide médical, si bien qu'une liaison fluidique pour l'amenée de liquide frais et l'évacuation de liquide usagé peut être établie.
